# EUROPÄISCHE PATENTANMELDUNG

(11) **EP 1 129 715 A1**
(43) Veröffentlichungstag der Anmeldung: **05.09.2001**
(21) Anmeldenummer: 00103956.9
(22) Anmeldetag: 25.02.2000
(51) Int. Cl.: A61K 33/06, A61P 3/00, A61P 9/00, A61P 19/00, A61P 35/00, A61P 17/00

(54) **Anwendung von Magnesium, Kalzium und Silizium zur Heilung verschiedener Krankheiten und zum allgemeinen Wohlbefinden**

(71) Anmelder: Reichen, Werner, Dr., 20354 Hamburg (DE); Meyer-Waarden, Ursula, 20354 Hamburg (DE)
(72) Erfinder: Reichen, Werner, Dr., 20354 Hamburg (DE); Meyer-Waarden, Ursula, 20354 Hamburg (DE)

(57) **Zusammenfassung**

Mineralien und Mineralsalze in nicht aktiviertem und aktivierten Zustand, die mindestens Magnesium, Kalzium und Silizium enthalten, oder Kombinationen davon und/oder Kombinationen mit anderen Mineralien, in nicht aktivierter und/oder aktivierter Form haben sich bei einer Reihe von Erkrankungen, u.a. bei Krebserkrankungen, Hauterkrankungen, Stoffwechselerkrankungen und - störungen, Herz- und Kreislauferkrankungen und Erkrankungen des rheumatischen Formenkreises als Inhibitoren des Krankheitsverlaufes und teilweise zur Rückbildung von Krebszellen und Metastasen erwiesen. Sie fördern zudem den Stressabbau und sind förderlich für das subjektive allgemeine Wohlbefinden beim Menschen und für das objektive allgemeine Wohlbefinden beim Tier.

## Beschreibung

Mineralien und Mineralsalze gehören zur Gruppe der kristallinen oder semi-kristallinen Produktegruppen. Es handelt sich um meist um mehr oder weniger wasserhaltige Kristalle der allgemeinen Formel A _{x/z}B_{w} ^{*} y H₂O, wobei A = ein- oder mehrwertiges Metall, H, (NH₄), etc., B = O, SO₄, CO₃ etc., z = Wertigkeit, x = 1.0 bis ca. 12, y = 0 bis ca. 8 und w = 1 bis ca. 4 ist. Charakteristisch für die meisten kristallinen Verbindungen ist, dass sie ihr Wasser stetig und ohne Änderung der Kristallstruktur abgeben, andere Verbindungen anstelle des Wassers aufnehmen und auch als lonenaustauscher und Katalysatoren wirken können.

Die Kristallgitter der Mineralien und Mineralsalze bauen sich aus verschiedenen geometrischen Strukturen , wie z.B. Tetraedern auf, die meistens über Sauerstoffbrücken verknüpft sind. Dabei entsteht eine räumliche Anordnung von gleich gebauten Absorptionshohlräume, die über Kanäle zugänglich sind. In ein derartiges Kristallgitter können in Abhängigkeit von der Porengrösse nur entsprechend kleine Moleküle eindringen. Mineralien und Mineralsalze können daher wirken wie ein Molekularsieb. Entsprechende technische Anwendungen sind bekannt (z. Reinigung von Gasen, Entfernung von Wasser aus organischen Lösungsmitteln usw.) Die zum Ausgleich der negativen Ladungen der einzelnen geometrischen Strukturen notwendigen Kationen sind im hydratisierten Gitter relativ leicht austauschbar. Diese Eigenschaften von Mineralien und Mineralsalzen wird z.B. in Waschmitteln zur Enthärtung (Bindung von Calcium) von Wasser ausgenutzt.

Mineralien und Mineralsalze können durch verschiedene Verfahren aktiviert werden wobei "Aktivierung" bedeutet, dass die Struktur des Kristallgitters partiell zerstört wird. Diese Zerstörung geschieht grundsätzlich durch hohe Energieeinwirkung auf die Kristalle und dadurch erfolgende asymmetrische Aufspaltung mit der Folge, dass neben den neutralen auch anionische, kationische und radikale Partikel entstehen.

Es sind verschiedene Studien durchgeführt worden und toxikologischen, pharmakologischen und klinischen Studien haben ergeben, dass Mineralien und Mineralsalze in nicht aktivierter und aktivierter Form verschiedene Krankheiten und Erkrankungen zu heilen vermögen. Die Art und Weise wie die Aktivierung dabei erfolgt, ist irrelevant.

Die Indikationen umfassen:
Karzinome jeglicher Art
Krebserkrankungen jeglicher Art
Hauterkrankungen
Multiple Sklerose
Alzheimer Krankheit
Rheumatische Krankheiten
Zystische Fibrose,
Virus-Infektionen
und weitere.

Toxikologische Studien mit Mineralien und Mineralsalzen haben ergeben, dass die LD₅₀ (= letale Halbwertsdosis) zwischen ca. 5 - 20% pro kg/KG (= Menge der Einnahme in Kilogramm pro Kilogramm Körpergewicht) liegt. Das heisst ,dass für einen Erwachsenen die Einnahme von ca. 2 - 20 kg (!) pro Tag mit einer 50%igen Wahrscheinlichkeit. tödlich sein können. Da die empfohlene Dosierung bei max. 14 g pro Tag liegt, wird die LD₅₀ um das zweihundertfünfzig bis tausendfache unterschritten, so dass die Unbedenklichkeit auch bei täglichem prophylaktischen Gebrauch gegeben ist. Da die kationische und anionische Austauschfähigkeit bei nicht aktivierten und aktivierten Mineralien und Mineralsalzen erwiesen ist, soll der Patentanspruch beide und alle Arten von Mineralien und Mineralsalzen, die Mindestens Magnesium, Kalzium und Silizium enthalten sowie alle Kombinationen davon und deren Kombiation mit anderen Mineralien und/oder Mineralsalzen umfassen.

Wir haben gefunden, dass Mineralien und Mineralsalze in nicht aktiviertem und aktivierten Zustand, die mindestens Magnesium, Kalzium und Silizium enthalten, oder Kombinationen davon und/oder Kombinationen mit anderen Mineralien, bei der Anwendung als Emulsion Hautkrankheiten, wie Neurodermitis, Akne, Ulcus cruris, Psoriasis, Decubitus, Herpes Zoster, Herpes Simplex und der Regenerierung von Brandwunden zu heilen vermag.

Wir haben gefunden, dass sich Mineralien und Mineralsalze in nicht aktiviertem und aktivierten Zustand, die mindestens Magnesium, Kalzium und Silizium enthalten, oder Kombinationen davon und/oder Kombinationen mit anderen Mineralien, zur Behandlung von Rheumaerkrankungen verschiedener Arten, wie z.B. rheumatische Arthritis, Arthrose, Spondylose und Diskopathie als wirksam erweist.

Wir haben gefunden, dass die Anwendung von Mineralien und Mineralsalzen in nicht aktiviertem und aktivierten Zustand, die mindestens Magnesium, Kalzium und Silizium enthalten, oder Kombinationen davon und/oder Kombinationen mit anderen Mineralien, Stoffwechselerkrankungen wie Diabetes mellitus, Gicht, Cholesterolämie und Triglyceridämie zu lindern oder zu beheben vermag.

Wir haben der heilsame Effekt von Mineralien und Mineralsalzen in nicht aktiviertem und aktivierten Zustand, die mindestens Magnesium, Kalzium und Silizium enthalten, oder Kombinationen davon und/oder Kombinationen mit anderen Mineralien, bei der Behandlung von Myopathie, Dystrophie, Atrophie und Osteoporose gefunden.

Wir haben die heilende Wirkung von Mineralien und Mineralsalzen in nicht aktiviertem und aktivierten Zustand, die mindestens Magnesium, Kalzium und Silizium enthalten, oder Kombinationen davon und/oder Kombinationen mit anderen Mineralien, auf Herz- und Kreislauferkrankungen, wie erweiterte Venen, Erkrankung der Herzkranzgefässe, Erkrankung von peripheren Gefässen gefunden.

Mineralien und Mineralsalze in nicht aktiviertem und aktivierten Zustand, die mindestens Magnesium, Kalzium und Silizium enthalten, oder Kombinationen davon und/oder Kombinationen mit anderen Mineralien, haben sich auch als wirksam erwiesen zum Stressabbau bei Mensch und Tier. Eine Beimischung von nicht aktivierten oder aktivierten Mineralien und Mineralsalzen, die Mindestens Magnesium, Kalzium und Silizium enthalten, oder Kombinationen davon und/oder Kombinationen mit anderen Mineralien, ins Schweinefutter während dreier Wochen vor dem Transport ins Schlachthaus hat gezeigt, dass die Herzinfarktrate bei den transportierten Tieren drastisch sinkt (um einen Faktor >10). Beim Menschen beeinflusst die Einnahme von Mineralien und Mineralsalzen in nicht aktiviertem und aktivierten Zustand, die mindestens Magnesium, Kalzium und Silizium enthalten, oder Kombinationen davon und/oder Kombinationen mit anderen Mineralien das allgemeine Wohlbefinden auch im Krankheitszustand wesentlich.

## Patentansprüche

1. Behandlung von Krebserkrankungen jeglicher Art, mit oder ohne Metastasen, mit aktivierten oder nicht aktivierten Mineralien und Mineralsalzen, die mindestens Magnesium, Kalzium und Silizium enthalten, oder Kombinationen davon und/oder Kombinationen mit anderen Mineralien, in Form von Pulver, Tabletten, Dragees, Filmtabletten, Kapseln, Trinklösungen oder -emulsionen und Injectabilia.

2. Behandlung von Hautkrankheiten jeglicher Art mit aktivierten oder nicht aktivierten Mineralien und Mineralsalzen, die mindestens Magnesium, Kalzium und Silizium enthalten, oder Kombinationen davon und/oder Kombinationen mit anderen Mineralien, in Form von Emulsionen, Lotionen, Salben, Crèmen, Sprays, Déodorants und aller möglichen galenischen Formen zur epikutanen und subkutanen Anwendung.

3. Behandlung von Stoffwechselerkrankungen wie Diabetes mellitus, Gicht, Cholesterolämie und Triglyceridämie mit aktivierten oder nicht aktivierten Mineralien und Mineralsalzen, die mindestens Magnesium, Kalzium und Silizium enthalten, oder Kombinationen davon und/oder Kombinationen mit anderen Mineralien, in Form von Pulver, Tabletten, Dragees, Filmtabletten, Kapseln, Trinklösungen oder -emulsionen und Injectabilia.

4. Behandlung von Herz- und Kreislauferkrankungen, wie erweiterte Venen, Erkrankung der Herzkranzgefässe, Erkrankung von peripheren Gefässen mit aktivierten oder nicht aktivierten Mineralien und Mineralsalzen, die mindestens Magnesium, Kalzium und Silizium enthalten, oder Kombinationen davon und/oder Kombinationen mit anderen Mineralien, in Form von Pulver, Tabletten, Dragees, Filmtabletten, Kapseln, Trinklösungen oder -emulsionen und Injectabilia.

5. Behandlung von Rheumaerkrankungen verschiedener Arten, wie z.B. rheumatische Arthritis, Arthrose, Spondylose und Diskopathie mit aktivierten oder nicht aktivierten Mineralien und Mineralsalzen, die mindestens Magnesium, Kalzium und Silizium enthalten, oder Kombinationen davon und/oder Kombinationen mit anderen Mineralien, in Form von Pulver, Tabletten, Dragees, Filmtabletten, Kapseln, Trinklösungen oder -emulsionen und Injectabilia.

6. Behandlung von Myopathie, Dystrophie, Atrophie und Osteoporose mit aktivierten oder nicht aktivierten Mineralien und Mineralsalzen, die mindestens Magnesium, Kalzium und Silizium enthalten, oder Kombinationen davon und/oder Kombinationen mit anderen Mineralien, in Form von Pulver, Tabletten, Dragees, Filmtabletten, Kapseln, Trinklösungen oder -emulsionen und Injectabilia.

7. Behandlung von Stress und/oder stressähnlichen Erscheinungen bei Mensch und Tier sowie die Vermittlung von subjektivem und/oder objektivem Wohlempfinden beim Menschen mit aktivierten oder nicht aktivierten Mineralien und Mineralsalzen, die mindestens Magnesium, Kalzium und Silizium enthalten, oder Kombinationen davon und/oder Kombinationen mit anderen Mineralien in Form von Pulver, Tabletten, Dragees, Filmtabletten, Kapseln, Trinklösungen oder -emulsionen und Injectabilia.
